# EUROPEAN PATENT APPLICATION

(11) **EP 1 820 474 A2**
(43) Date of publication of application: **22.08.2007**
(21) Application number: 07109523.6
(22) Date of filing: 09.09.2002
(51) Int. Cl.: A61F 2/16

(54) **Intraocular lens extracting device**

(30) Priority: 07.09.2001 US 294065 P
(62) Divisional of application: 02759602.2
(71) Applicant: Siepser, Steven B., Paoli, PA 19301 (US)
(72) Inventor: Siepser, Steven B., Paoli, PA 19301 (US)
(74) Representative: Molnia, David

(57) **Abstract**

The present invention relates generally to a medical device, and more particularly to an ophthalmic surgical device for extracting an intra-ocular lens implant.

## Description

### Related Application

This application claims the benefit of U. S. Provisional Application No. 60/294,065, filed on September 7, 2001, which is incorporated here in its entirety.

### Field of the Invention

The present invention relates generally to a medical device, and more particularly to an ophthalmic surgical device for extracting an intra-ocular lens implant.

### Background of the Invention

In the United States there are 1,500, 000 cataract extractions a year, making this procedure one of the most commonly performed surgery. The complication rate is believed to be about 2%, which would be 30,000 cases a year of an intraocular mishap. There is probably a 20% incidence in this group of complications that are repairable by removing and replacing the intra-ocular lens implant (implant). This necessitates the removal of approximately 6,000 implants a year with no standardized systems or management organization to remove these lenses.

Cataracts are the leading cause of blindness in the world. The clouding of the human lens causes cataracts. In reality the human lens yellows and becomes less clear with age, when it clouds it is called a cataract. The cataract is somewhat akin to water that appears clear until it goes over a waterfall and appears white to the observer. In fact the word cataract is derived from the same Greek root for the name of a waterfall or cataract.

The human lens is responsible for focusing the images on the back of the eye that are in the gaze of the eye. The human eye is somewhat analogous to a simple film camera. There are some lenses that focus the light at the front of the camera as in the eye. This focusing the light on the film or in the case of the eye the retina, which is a neurosensory membrane, that converts the optical image into neurological electrical activity that is transmitted to the vision center of the brain. If one thinks of the eye as a video camera that has a chip (retina) that converts the image to electrical activity and send it to the monitor which in the eye's case is the occipital lobe of the brain.

Cataract extraction results from removing the cloudy lens from the eye. This also causes the eye to loose its ability to focus light on the retina unless it is replaced. In days gone by the elderly cataract patient was relegated to a handicapped life only being able to see with extremely thick aphakic (no lens) glasses. In the recent past, patients had to spend 2 weeks in the hospital with sand bags around their head and never returned to a fully active life again due to the distortion of these glasses and the magnification and distortion issues, even with contact lenses.

In the last 20 years this has all been changed because the human lens is now replaced with a plastic intra-ocular implant. This allows the eye to return to its more normal vision by returning the eye to a near physiologic or normal status. Every eye has different focusing powers because they differ in size and shape, like all human characteristics. Therefore each eye must have its power determined and calculated so that the correct replacement implant diopters power can be implanted. This measurement is done using ultrasound A-scan that measure the length of the eye much like a depth finder on a boat, that measures the depth of the water below the boat.

By using regression formulas the power can be fairly accurately determined. The problem is that various errors can be introduced resulting in the placement of the incorrect power lens. Certain implants may not tolerated by the patient and must be replaced to clear their vision due to; the wrong power lens being implanted, the lens becoming displaced, or the patient is experiencing glare. There have been increasing numbers of exchanges over the recent years as patient expectations rise and the ability to get better visual results.

This led to the development of a device that easily and safely removes implants. Making them easier to remove and carefully replaced with the proper lens.

### Summary of the Invention

The present invention, the Siepser Splitter, is an intraocular device designed to cut or section an intra-ocular lens in the eye in order to remove it through the original small implantation incision. The device represents a departure from all present technology, which makes use of either a scissor or a snare like mechanism. The Siepser Splitter uses a unique basis for operation. Present scissor technology with ancient scissor action tend to induce what can be described as the watermelon seed effect, when the jaws of the scissor squeezes the implant it tends to shoot forward away from the jaws. Attempts to correct this problem have been to serrate the jaws to better grab the lens and hold it in place.

Snare based technology exists, that uses a wire to grasp and then transect the implant much like a wire cheese cutter. This transects the lens but causes it to buckle and bunch up during the cutting process and this can cause damage to the eye as the lens springs back to its normal shape and can damage delicate structures of the eye.

One advantage of the splitter is that it holds the implant and drives it into a sharp wedge that splits it for removal with a controlled action.

The device has some of the characteristics of a hydraulic wood splitter. The main difference from present technology is that the Siepser splitter grabs the lens, holds it in position and gently sections it in half for removal from the eye. It can be made in a disposable or reusable model.

### Brief Description Of The Drawings

For the purpose of illustrating the invention, there is shown in the drawings a form, which is presently preferred; it being understood, however, that this invention is not limited to the precise arrangements and instrumentalities shown.

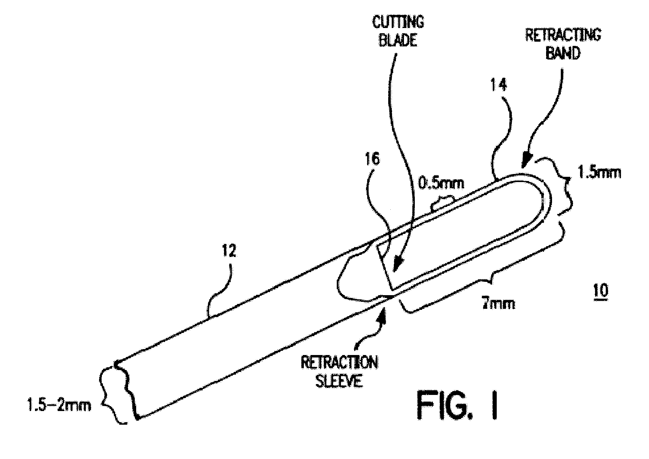

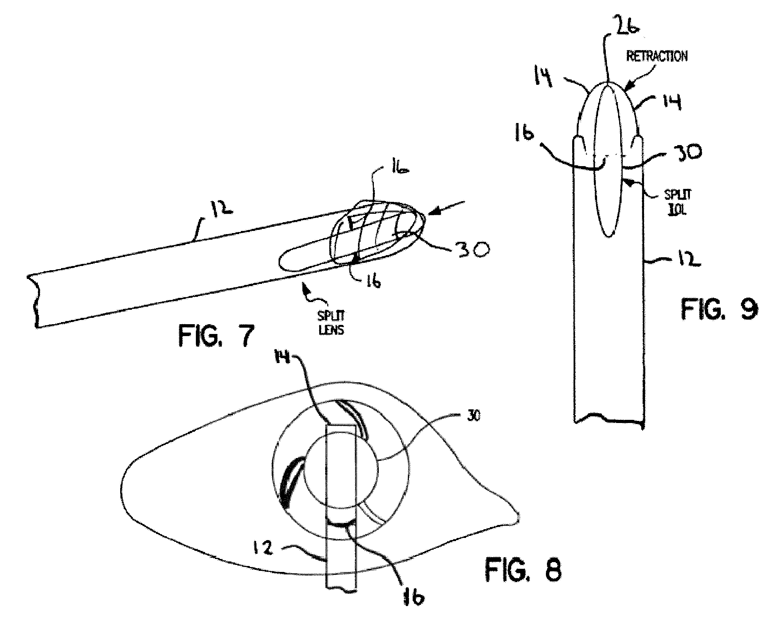

Figures 1 through 8 illustrate an extraction device for a deformable intraocular lens according to one embodiment of the present invention.

### Detailed Description Of The Invention

Referring now to the drawings, wherein like numerals indicate like elements, there is shown in FIG.1 an example of an extraction device 10 for extracting a deformable intraocular lens.

The extraction device 10 has a body 12 with a retracting band 14 that is movable within the body 12 and a cutting blade 16. The retracting band 14 is moved so that it extends longitudinally out of the body 12 in order to engage an implanted deformable intraocular lens 30. The retracting band 14 is extended out of the body 10 to engage and hold the lens 30 and draw it towards the cutting blade 16. As the lens 30 is drawn into the cutting blade 16, the lens is split allowing it to be safely retracted from the posterior chamber.

In one aspect of the invention the body 12 has a cylindrical or tube like shape. The mechanism that moves the retracting band 14 is hand or finger actuated, and may include a plunger, screw, plier or lever, or a sliding slot action. The mechanism may use a cross or cam action to provide controlled unidirectional movement of the retracting band 14.

In another aspect of the invention, the retracting band 14 is a wire or a flattened loop that is wide enough to grasp the implant without cutting it. The retracting band 14 has a width between about. 1mm and3mm, and is may made from plastic, rubber, silicone, acrylic, Teflon, metal, titanium, surgical steel, or high carbon steel. The retracting band 14 is about 4-15 mm in length or longer as required by the material selected or type and size of intraocular lens to be extracted. The cutting blade 16 can be a flat or preferable a wedge shaped cutting blade.

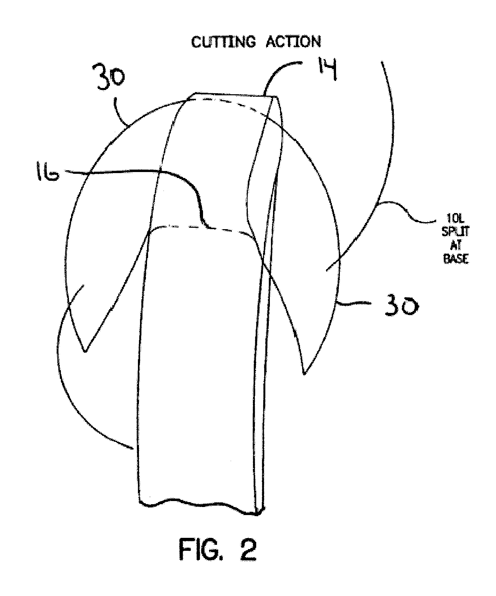

As shown in FIG. 2, the retracting band 14 captures and draws the lens 30 into the wedged shaped cutting blade16 splitting the lens 30 akin to the action of a hydraulic wood splitter. In this context, instead of splitting the lens by driving a splitting tool into the material of the lens-which would be very ineffective since most lenses tend to slip away from such cutting methods-the present invention retains the lens 30 and slowly pushes it against an anvil like splitting wedge 16 that slowly and effectively cuts the lens 30. In addition to cutting the lens 30, the retracting band 14 stabilizes the lens 30 as it draws it into the cutting wedge 16.

The retracting band 14 can be made from a single piece of material or from welded components and is connected to an actuating plunger or rod. The rod can be moved in the handle to actuate the retracting band 14.

The mechanism for moving the retracting band 14 can be variable. A simple pull plunger can be grasped by the second hand to pull the retracting band forward. A single handed version can be made using the same technology with the retracting band 14 affixed to the base of the body 12 and the body 12 may itself be advanced over the retracting band 14 by sliding the body 12 back and forward over the retracting band 14.

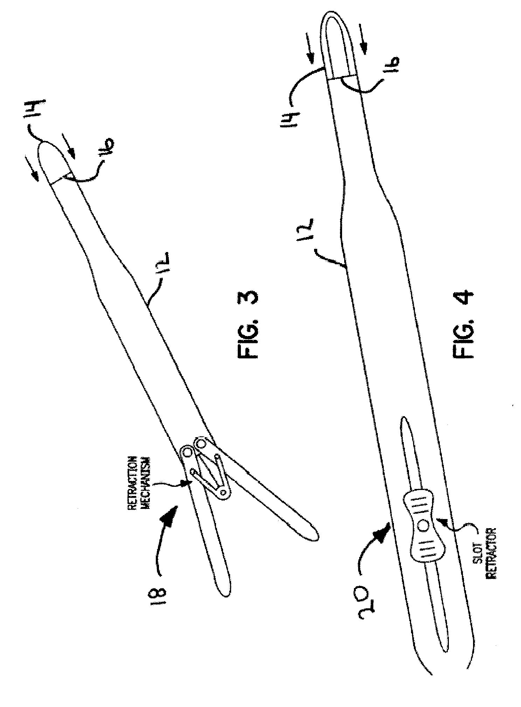

As shown in FIG. 3, a bi-hinged scissor-like mechanism 18 can be used that pulls the retracting band 14 forward by compressing itself and extending out the end attached to the retracting band 14 that advances it in the tube-like body 14. A screw-type action [not shown] device can also be used. For example, by twisting a knob at the base of the instrument the retracting band can be advanced on a screw that pulls it along. As shown in FIG. 4, a slot retractor action 20 can also be used to effect the movement of the retracting band 14.

The mechanical action retracting band 14can also be developed by a cam- type action [not shown]. For example, the retracting band is attached to a pin in a cam that can be rotated drawing the retracting band toward. Also a crankshaft- offset action [not shown] can also be used to increase the throw and therefore the movement of the rod attached to the retracting band.

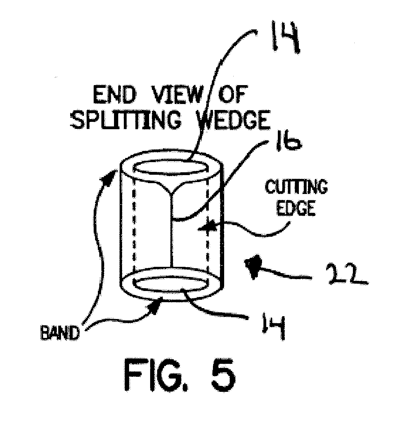

As shown in FIG. 5, the cutting surface 16 on the wedge 22 can be sharpened plastic or steel. It can be a separate piece of surgical steel or specially prepared blade surface to cut and split the implant as it is drawn on to the blade area. The cutting blade 16 can be vertical, horizontal, or slanted in a way to increase the slicing effect of the cutting blade 16. 1 may be curved or v shaped to assist in stabilizing and easing the cutting action of the cutting blade 16.

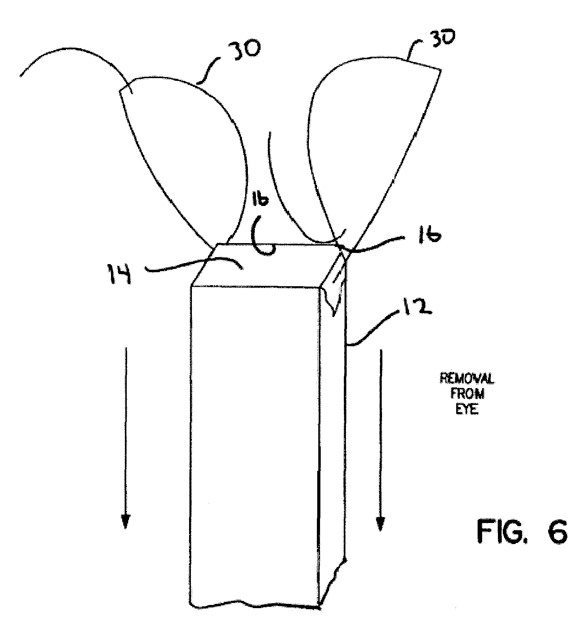

As shown in FIG. 6, a shoulder behind or as part of the cutting blade 16 helps to direct the cut pieces away from the delicate structures of the eye. The sluiceway directs the cut pieces and controls there movement. The retracting band 14 can be set so as not to fully transect the lens 30 allowing the two pieces to be more easily removed from the eye in one movement.

As shown in FIG. 's 7 through 9, an another aspect of the invention includes a split or two piece retraction band 14. In this aspect the bands are split at the tip 26 of the retraction band 14. As the bands are extend out from the body 12 they separate to provide longitudinal access to the tens 30 without the need to angle the device 10 and avoid further disruption to the incision site.

These and other advantages of the present invention will be apparent to those skilled in the art from the foregoing specification. Accordingly, it will be recognized by those skilled in the art that changes or modifications may be made to the above-described embodiments without departing from the broad inventive concepts of the invention. It should therefore be understood that this invention is not limited to the particular embodiments described herein, but is intended to include all changes and modifications that are within the scope and spirit of the invention as set forth in the claims.

## Claims

1. An intraocular lens cutter, comprising:
a body for engaging a portion of a deformable intraocular lens; and
a cutting blade for engaging a distal portion of the lens from that engaged by the body, wherein upon the application of force, the cutting blade cuts the lens.

2. The intraocular lens cutter of claim 8, wherein the body is of a width sufficient to grasp the lens without cutting it.

3. The intraocular lens cutter of claim 8, wherein the body is two piece.

4. The intraocular lens cutter of claim 8, wherein the body surrounds the sides of the cutting blade during dissection of the lens.

5. The intraocular lens cutter of claim 8, further comprising a plunger for providing said application of force.

6. The intraocular lens cutter of claim 8, wherein the body has a curved tip adapted to engage a portion of a deformable intraocular lens.

7. An intraocular lens cutter, comprising:
a band for engaging a portion of a deformable intraocular lens; and
a cutting blade for engaging a distal portion of the lens from that engaged by the band, wherein upon the application of force, the cutting blade cuts the lens.

8. The intraocular lens cutter of claim 14, wherein the band is of a width sufficient to grasp the lens without cutting it.

9. The intraocular lens cutter of claim 14, wherein the band has a curved tip and the arc defined by the curved tip has a diameter that is between about 1mm to about 3 mm.

10. The intraocular lens cutter of claim 14, wherein the band is two piece.

11. The intraocular lens cutter of claim 14, wherein the band surrounds the sides of the cutting blade during dissection of the lens.

12. The intraocular lens cutter of claim 14, wherein the cutter comprises a body and the band is comprised in the body.

13. The intraocular lens cutter of claim 14, further comprising a plunger for providing said application of force.

14. The intraocular lens cutter of claim 14, wherein the cutting blade is wedge shaped.

15. The intraocular lens cutter of claim 14, wherein the band defines an opening for engaging a portion of a deformable intraocular lens.

16. An intraocular lens cutter, comprising:
a capture portion for engaging a deformable intraocular lens;
a cutting portion, spaced apart from the capture portion along a common axis to allow the lens to be disposed therebetween; and
means for creating movement between the capture portion and the cutting portion to exert force upon the lens between them, thereby sectioning the lens in half.

17. The intraocular lens cutter of claim 23, wherein the capture portion has a curved tip.

18. The intraocular lens cutter of claim 23, wherein the capture portion is two piece.

19. The intraocular lens cutter of claim 23, wherein the capture portion surrounds the sides of the cutting portion during dissection of the lens.

20. The intraocular lens cutter of claim 23, wherein the means provide plunger, screw, plier, lever, sliding slot, or cam action.

21. A single use disposable extraction device kit, comprising:
intraocular lens cutter of claim 8; and
packaging for the cutter.

22. A single use disposable extraction device kit, comprising:
intraocular lens cutter of claim 14; and
packaging for the cutter.

23. A single use disposable extraction device kit, comprising:
intraocular lens cutter of claim 23; and
packaging for the cutter.

24. A reusable extraction device kit, comprising:
intraocular lens cutter of claim 8; and
packaging for the cutter.

25. A reusable extraction device kit, comprising:
intraocular lens cutter of claim 14; and
packaging for the cutter.

26. A reusable extraction device kit, comprising:
intraocular lens cutter of claim 23; and
packaging for the cutter.

27. The intraocular lens cutter of claim 8, wherein said body defines a recess for receiving a portion of a deformable intraocular lens.

28. The intraocular lens cutter of claim 8, wherein said body defines an opening for receiving a portion of a deformable intraocular lens.

29. The intraocular lens cutter of claim 8, wherein a distal end of said body is adapted to engage a portion of a deformable intraocular lens.

30. The intraocular lens cutter of claim 14, wherein said band defines a recess for receiving a portion of a deformable intraocular lens.
